# EUROPÄISCHE PATENTSCHRIFT

(11) **EP 0 329 944 B1**
(45) Veröffentlichungstag und Bekanntmachung des Hinweises auf die Patenterteilung: **18.11.1993**
(21) Anmeldenummer: 89100855.9
(22) Anmeldetag: 19.01.1989
(51) Int. Cl.: A61B 17/32

(54) **Gerät mit einem rotierend angetriebenen chirurgischen Instrument**
Device with a rotary driven surgical instrument
Appareil avec un instrument chirurgical entrainé en rotation

(30) Priorität: 19.02.1988 DE 3805179
(43) Veröffentlichungstag der Anmeldung: 30.08.1989
(73) Patentinhaber: Richard Wolf GmbH, 75438 Knittlingen (DE)
(72) Erfinder: Ams, Felix, Dipl.-Ing., D-7539 Kämpfelbach (DE); Baier, Manfred, D-7518 Bretten (DE); Schäfer, Roland, D-7518 Bretten-Diedelsheim (DE)
(74) Vertreter: Wilcken, Thomas, Dipl.-Ing.

(56) Entgegenhaltungen:
- EP-A- 0 189 807
- EP-A- 0 253 478
- GB-A- 2 042 902

## Beschreibung

Die Erfindung betrifft ein Gerät mit einem rotierend angetriebenen chirurgischen Instrument mit einem Handstück mit einer in diesem drehbar gelagerten Halterung zur Aufnahme von auswechselbaren Schneid- und Fräseinsätzen, einem Antriebsmotor zum Antreiben der die Werkzeugeinsätze aufnehmenden Halterung, einer Steuereinheit zur Steuerung des Antriebsmotors mit Mitteln zum Einstellen der Drehzahl und Vorwählen des Drehzahlbereiches und der Drehrichtung sowie einer digitalen Anzeige zum Ablesen der jeweils eingestellten Drehzahl.

Aus der DE-C-2 848 314 ist ein chirurgisches Instrument für die arthroskopische intra-artikuläre Gelenkchirurgie bekannt, welches, ohne eine Gelenköffnung vornehmen zu müssen, beispielsweise das Abtragen von Synovialgewebe, eine partielle Meniskusresektion, die Behandlung von Knorpeldefekten oder dergleichen durch einen endoskopischen Eingriff ermöglicht. Die Drehzahl der in einem Außenschaft rotierenden Schneid- oder Fräseinsätze kann an dem mit dem Instrument über eine flexible Welle verbundenen Antriebsmotor im Bereich von 100 bis 200 Umdrehungen pro Minute eingestellt werden. Ferner besteht die Möglichkeit, die abgetragenen Gewebe-, Knorpel- oder Knochenteile durch den hohlen Schneid- oder Fräseinsatz hindurch mittels einer am Instrument anschließbaren Unterdruckleitung abzufördern.

Aus der EP-A2 0 189 807 ist weiter ein chirurgisches Instrument bekannt, welches eine Mehrzahl von Adaptern aufweist, die zwischen dem Motorhandgriff und dem Schneideinsatz angeordnet die Drehzahl des gerade verwendeten Schneid- oder Fräseinsatzes einstellen. Da die verschiedenen Schneideinsätze mit unterschiedlichen Drehzahlen zu betreiben sind, erfordert dies, daß jedem der Einsätze ein Adapter zugeordnet wird.

Schließlich ist ein unter der Bezeichnung INTRA-ARC DRIVE SYSTEM von der Firma Concept vertriebenes chirurgisches Instrument für die arthroskopische Behandlung von Gelenken bekannt. Die Anordnung einer Vielzahl von am distalen Handgriffende befindlichen Tastern ermöglicht zwar,das Instrument ein- und auszuschalten, die Drehrichtung zu ändern sowie die Drehzahl in zwei Drehzahlbereichen einzustellen. Da derartige Instrumente jedoch in der Regel mehreren Anwendern zur Verfügung stehen, die in Abhängigkeit des durchzuführenden chirurgischen Eingriffes, des verwendeten Schneideinsatzes und der Erfahrung verschiedene Drehzahlen wählen, haftet dem bekannten Stand der Technik der Mangel an, daß bei jeder Benutzung des Instrumentes und bei jedem Austausch des Schneideinsatzes die nach Ansicht des jeweiligen Anwenders optimale Drehzahl neu eingestellt werden muß. Die zur Ermittlung der optimalen Drehzahl erforderliche Zeit und auch die Ermittlung der optimalen Drehzahl selbst stellen sowohl für den Chirurgen als auch für den Patienten eine unzumutbare Belastung dar.

Die Aufgabe der Erfindung besteht daher darin, ein Gerät der einleitend angeführten Art mit einer Steuereinheit für den Antrieb eines chirurgischen Instrumentes so zu verbessern, um individuelle drehzahlbezogene Einstelldaten abrufbar speichern zu können.

Diese Aufgabe wird erfindungsgemäß durch die Merkmale des Patentanspruchs 1 gelöst.

Eine bevorzugte Ausgestaltung besteht darin, daß der Codeträger lösbar mit der Steuereinheit verbindbar ist, was vorteilhafterweise dadurch ermöglicht ist, daß der Codeträger z.B. als Karte, Schlüssel, Stecker ausgebildet ist.

Dadurch können einmal als günstig ermittelte und erprobte Einstelldaten in ihrer Gesamtheit gespeichert und auf einen Codeträger übertragen werden, der jederzeit eine individuelle Einstellung bewirkt, wenn dieser mit der Decodiereinrichtung in Wirkverbindung gebracht wird.

Für die Aktivierung und Deaktivierung der Steuereinheit kann dieser Einheit eine fußbetätigbare Schalteinrichtung zum Ein- und Ausschalten des Antriebsmotors und Ändern der Drehrichtung desselben zugeordnet sein. Zu diesem Zweck kann die Schalteinrichtung zum Ein- und Ausschalten des Antriebsmotors und Ändern der Drehrichtung desselben alternativ dem Handstück zugeordnet sein. Die verschiedenen, zur Anwendung kommenden Schneid- und Fräseinsätze sind zwecks Entfaltung ihrer Wirksamkeit in spezifischen Drehzahlbereichen zu betreiben. Mit Hilfe einer dem jeweils benötigten Drehzahlbereich entsprechenden Codierung der Schneid- und Fräseinsätze kann eine werkzeugspezifische, automatische Drehzahlbereichs-Vorwahl dadurch erreicht werden, daß das Handstück eine Einrichtung zur Umwandlung der den Schneid-und Fräseinsätzen zugeordneten Codes in ein elektrisches Signal aufweist, welches durch die Steuereinheit ausgewertet wird. Dabei kann die Codierung mechanischer, elektrischer, optischer oder magnetischer Art sein, so daß die Decodiereinrichtung in dem Handstück entsprechend ausgeführt ist.

Zur Erzielung einer effektiveren Schneidleistung kann in Abhängigkeit des verwendeten Schneidwerkzeuges die Steuereinheit so ausgelegt sein, daß sie eine Betriebsart ermöglicht, bei der die Drehrichtung des Antriebsmotors periodisch wechselt, wobei die Umschaltung beispielsweise jeweils nach Ablauf von mindestens einer vollständigen Umdrehung erfolgt. Eine weitere Vereinfachung der Bedienbarkeit des erfindungsgemäßen Instrumentes läßt sich dadurch erzielen, daß die Steuereinheit eine Spracheingabeeinrichtung aufweist, die eine Funktionssteuerung über Sprache ermöglicht. Schließlich kann die Steuereinrichtung zum Zwecke der Dokumentation eine Anschlußmöglichkeit aufweisen, mit welcher die eingestellten Daten in ein externes Aufzeichnungsgerät übertragen und/oder auf einen Monitor beispielsweise in ein Endoskopbild eingeblendet werden können.

Das erfindungsgemäße Gerät wird nachfolgend anhand eines in der Zeichnung dargestellten Ausführungsbeispieles näher erläutert. Es zeigt:

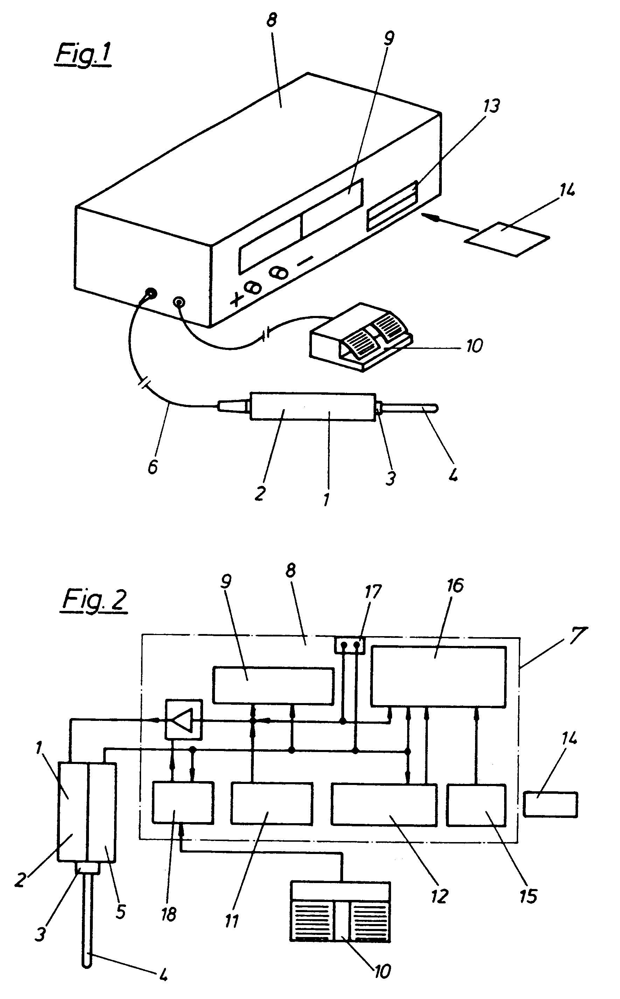

- Figur 1: eine schematische Ansicht des kompletten Gerätes und
- Figur 2: das Gerät in schematischer Darstellung als Blockschaltbild.

Wie aus den Figuren ersichtlich, weist das Handstück 1 einen Antriebsmotor 2 auf, der eine die Schneid- und Fräseinsätze 4 aufnehmende Halterung 3 antreibt, sowie einen Decoder 5 zur Umwandlung der jeweils den Schneid- und Fräseinsätzen 4 zugeordneten Codes in entsprechende elektrische Signale. Das Handstück 1 ist über ein Kabel 6 mit einer Steuereinheit 7 verbunden, welche in einem geeigneten Gehäuse 8 untergebracht ist. Äußerlich sichtbare Elemente dieser Steuereinheit 7 sind ein Display 9 zur Anzeige der eingestellten Drehzahl und beispielsweise der verwendeten Werkzeugart,ein Anschluß für einen Fußschalter 10 zum Aktivieren und Deaktivieren sowie zum Umsteuern der Drehrichtung des Antriebsmotors 2, eine Regeleinrichtung 11 zur Einstellung der Drehzahl, ein Drehzahl-Bereichswähler 12 sowie eine Steckfassung 13 für einen Codeträger 14. Die Steckfassung 13 steht mit einem Coder/Decoder 15 in Verbindung, der wiederum mit einer Speichereinheit 16 korrespondiert.

Zur Vorbereitung des Betriebes des erfindungsgemäßen Gerätes nimmt der jeweilige Benutzer eine manuelle Einstellung der Betriebsdaten,wie Drehzahl,Anfangs- und Endwert derselben u.s.w.,vor und optimiert diese nach seinem Empfinden. Die so gefundenen Betriebsdaten werden in der Speichereinheit 16 gespeichert, die zu diesem Zweck eine entsprechende Anzahl Speicherplätze aufweist. Die Inhalte dieser Speicherplätze lassen sich auf einen Codeträger 14, beispielsweise in Form einer Magnetkarte, durch Einstecken derselben in die dafür vorgesehene Steckfassung 13 übertragen. Damit ist umgekehrt der Besitzer dieses Codeträgers in der Lage, die vorprogrammierten Daten durch Einstecken der Magnetkarte aufzurufen, das heißt, die Steuereinheit 7 durch diesen Vorgang auf die entsprechenden Daten einzustellen. Dabei kann im Falle der Verwendung von in der oben erwähnten Weise kodierten Werkzeugeinsätzen ein Drehzahlbereich vorgegeben werden, innerhalb dessen oberer und unterer Drezahlgrenze eine manuelle Nachregelung vorgenommen werden kann. Es kann so jeder Benutzer einen individuellen Codeträger benutzen, mittelswelchem durch einfaches Einstecken desselben in die Steckfassung 13 das Gerät jederzeit auf die von ihm beispielsweise auf empirischem Wege ermittelten und als optimal empfundenen Daten eingestellt werden kann. Das so eingestellte Gerät läßt sich nun durch Betätigen des Fußschalters 10 über die Drehrichtungssteuerung 18 aktivieren oder deaktivieren, wobei auch die Drehrichtung über denselben eingestellt werden kann.

Die Einstelldaten können über eine geeignete Schnittstelle 17 auf ein externes Aufzeichnungsgerät übertragen werden, mit dessen Hilfe eine Speicherung des Eingriffsvorganges beispielsweise zu Lehrzwecken oder zum Zwecke der Dokumentation möglich ist. Es kann der Eingriff aber auch durch Anschluß eines Monitors extern verfolgt werden, der beispielsweise das endoskopisch erzeugte Bild des Operationsfeldes wiedergibt, in das die Einstelldaten eingeblendet werden.

Abschließend wird noch darauf hingewiesen, daß auf dem jeweiligen Codeträger nur der personenbezogene Code des Anwenders vermerkt sein kann, während die zu speichernden Daten, wie Drehzahlen, Drehrichtung, Drehzahlbereiche und dergleichen, im internen Systemspeicher abgelegt werden und über den Codeträger abgerufen werden können. Andererseits können die zu speichernden und anwenderbezogenen Daten auch unmittelbar auf dem Codeträger abgespeichert werden, so daß diese Daten beim Einstecken des Codeträgers in das Gerät eingelesen und auf den internen Systemspeicher übertragen werden. In diesem Fall ist übrigens ein gesonderter personenbezogener Code nicht erforderlich.

## Patentansprüche

1. Gerät mit einem rotierend angetriebenen chirurgischen Instrument mit einem Handstück (1) mit einer in diesem drehbar gelagerten Halterung (3) zur Aufnahme von auswechselbaren Schneid- und Fräsereinsätzen (4), einem Antriebsmotor (2) zum Antreiben der die Werkzeugeinsätze (4) aufnehmenden Halterung (3), einer Steuereinheit (7) zur Steuerung des Antriebsmotors (2) mit Mitteln zum Einstellen der Drehzahl und Vorwählen des Drehzahlbereiches und der Drehrichtung sowie einer Anzeige zum Ablesen der jeweils eingestellten Drehzahl, dadurch gekennzeichnet, daß die Steuereinheit (7) einen nichtflüchtigen Schreib-Lese-Speicher (16) zur digitalen Speicherung von anwenderspezifischen Drehzahldaten, einen Codeträger (14) für einen personenbezogenen Code, dem in dem Schreib/Lese-Speicher (16) individuelle anwenderspezifische Daten zugeordnet sind, oder für gespeicherte anwenderspezifische Daten sowie eine Codier-Decodiereinrichtung (15) aufweist.

2. Gerät nach Anspruch 1, dadurch gekennzeichnet, daß der Codeträger (14) mit der Steuereinheit (7) lösbar verbindbar ist.

3. Gerät nach den Ansprüchen 1 oder 2, dadurch gekennzeichnet, daß der Codeträger (14) als Karte, Schlüssel oder Stecker ausgebildet ist.

4. Gerät nach einem der Ansprüche 1 bis 3, dadurch gekennzeichnet, daß der Steuereinheit (7) eine fußbetätigbare Schalteinrichtung (10) zum Ein- und Ausschalten des Antriebsmotors (2) und Ändern der Drehrichtung desselben zugeordnet ist.

5. Gerät nach einem der Ansprüche 1 bis 3, dadurch gekennzeichnet, daß eine fußbetätigbare Schalteinrichtung (10) zum Ein- und Ausschalten des Antriebsmotors (2) und Ändern der Drehrichtung desselben dem Handstück (1) zugeordnet ist.

6. Gerät nach einem der Ansprüche 1 bis 5, dadurch gekennzeichnet, daß das Handstück (1) einen Decoder (5) zur Umwandlung eines den Schneid- und Fräseinsätzen (4) zugeordneten Codes in ein elektrisches Signal aufweist, welches durch die Steuereinheit (7) ausgewertet wird.

7. Gerät nach einem der Ansprüche 1 bis 6, dadurch gekennzeichnet, daß die Steuereinheit (7) in Abhängigkeit des Schneidwerkzeuges eine Betriebsart ermöglicht, bei der die Drehrichtung des Antriebsmotors (2) periodisch wechselt, wobei die Umschaltung jeweils nach Ablauf mindestens einer vollständigen Umdrehung erfolgt.

8. Gerät nach einem der Ansprüche 1 bis 7, dadurch gekennzeichnet, daß die Steuereinheit (7) eine Spracheingabeeinrichtung aufweist, die eine Funktionssteuerung über Sprache ermöglicht.

9. Gerät nach einem der Ansprüche 1 bis 8, dadurch gekennzeichnet, daß die Steuereinrichtung (7) eine Anschlußstelle (17) aufweist, über welche die eingestellten Betriebsdaten in ein externes Aufzeichnungsgerät übertragen und/oder auf einem Monitor, beispielsweise in ein Endoskopbild, eingeblendet werden.

## Claims

1. Implement with a rotatably driven surgical instrument with a handpiece (1) with a mounting (3) rotatably supported therein to hold interchangeable cutting and milling inserts (4), with a driving motor (2) to drive the mounting (3) holding the tool inserts (4), with a control unit (7) to control the driving motor (2) with means to set the rotational speed and preselect the rotational speed range and the direction of rotation and also with a display to read the rotational speed set at any given time, characterised in that the control unit (7) has a non-volatile write/read memory (16) for the digital storage of user specific rotational speed data, a code carrier (14) for a person-related code, with which in the write/read memory (16) individual user-specific data are associated, or for stored user-specific data and also has a coding/decoding device (15).

2. Implement according to Claim 1, characterised in that the code carrier (14) is detachably connectable with the control unit (7).

3. Implement according to Claims 1 or 2, characterised in that the code carrier (14) is constructed as a card, key or plug.

4. Implement according to one of Claims 1 to 3, characterised in that there is associated with the control unit (7) a foot-operable switching device (10) to switch the driving motor (2) on and off and to alter the direction of rotation thereof.

5. Implement according to one of Claims 1 to 3, characterised in that a foot-operable switching device (10) is associated with the handpiece (1) to switch the driving motor (2) on and off and to alter the direction of rotation thereof.

6. Implement according to one of Claims 1 to 5, characterised in that the handpiece (1) has a decoder (5) to convert a code, associated with the cutting- and milling inserts (4), into an electric signal, which is evaluated by the control unit (7).

7. Implement according to one of Claims 1 to 6, characterised in that the control unit (7) makes possible, depending on the cutting tool, a type of operation in which the direction of rotation of the driving motor (2) changes periodically, in which the changeover takes place at any given time after the completion of at least one complete revolution.

8. Implement according to one of Claims 1 to 7, characterised in that the control unit (7) has a speech input device, which makes possible the functional control by means of speech.

9. Implement according to one of Claims 1 to 8, characterised in that the control unit (7) has a connection site (17), via which the set operating data are transferred into an external recording apparatus and/or are faded in on a monitor, for example in an endoscope image.

## Revendications

1. Appareil comprenant un instrument chirurgical entraîné en rotation comportant une pièce formant poignée (1) dans laquelle est monté tournant un support (3) destiné à recevoir des inserts de coupe et de fraisage (4) interchangeables, un moteur d'entraînement (2) pour entraîner le support (3) recevant les inserts d'outils (4), une unité de commande (7) pour commander le moteur d'entraînement (2) et comportant des moyens pour régler la vitesse de rotation et présélectionner la plage de vitesses de rotation et le sens de rotation, ainsi qu'un dispositif d'affichage permettant chaque fois la lecture de la vitesse de rotation réglée, caractérisé en ce que l'unité de commande (7) comporte une mémoire à lecture-écriture non volatile (16) pour la mémorisation numérique de données de vitesse de rotation spécifiques à l'utilisateur, un support de code (14) pour un code personnel, auquel sont affectées des données individuelles spécifiques à l'utilisateur dans la mémoire à lecture-écriture (16), ou pour des données spécifiques à l'utilisateur mémorisées, ainsi qu'un dispositif de codage-décodage (15).

2. Appareil selon la revendication 1, caractérisé en ce que le support de code (14) peut être relié à l'unité de commande (7), de manière amovible.

3. Appareil selon les revendications 1 ou 2, caractérisé en ce que le support de code (14) est réalisé sous forme de carte, clé ou fiche.

4. Appareil selon l'une des revendications 1 à 3, caractérisé en ce qu'à l'unité de commande (7) est associé un dispositif de commutation (10) pouvant être actionné au pied, pour la mise en marche et l'arrêt du moteur d'entraînement (2) et pour modifier le sens de rotation de celui-ci.

5. Appareil selon l'une des revendications 1 à 3, caractérisé en ce qu'un dispositif de commutation (10) pouvant être actionné au pied, pour la mise en marche et l'arrêt du moteur d'entraînement (2) et pour modifier le sens de rotation de celui-ci, est associé à la pièce formant poignée (1).

6. Appareil selon l'une des revendications 1 à 5, caractérisé en ce que la pièce formant poignée (1) comporte un décodeur (5) pour convertir un code associé aux inserts de coupe et de fraisage (4), en un signal électrique, qui est évalué par l'unité de commande (7).

7. Appareil selon l'une des revendications 1 à 6, caractérisé en ce que l'unité de commande (7), en fonction de l'outil de coupe, permet un mode de fonctionnement selon lequel le sens de rotation du moteur d'entraînement (2) s'inverse périodiquement, chaque commutation s'effectuant après au moins un tour complet.

8. Appareil selon l'une des revendications 1 à 7, caractérisé en ce que l'unité de commande (7) comporte un dispositif d'entrée vocale, qui permet une commande de fonctionnement à la voix.

9. Appareil selon l'une des revendications 1 à 8, caractérisé en ce que le dispositif de commande (7) comporte une interface de raccordement (17) par l'intermédiaire de laquelle les données de fonctionnement réglées peuvent être transmises à un dispositif d'enregistrement externe et/ou être affichées en surimpression sur un moniteur, par exemple sur une image d'endoscope.
